# EUROPEAN PATENT SPECIFICATION

(11) **EP 1 495 763 B1**
(45) Date of publication and mention of the grant of the patent: **18.03.2009**
(21) Application number: 02718592.5
(22) Date of filing: 15.04.2002
(51) Int. Cl.: A61K 36/00, A61K 31/10, A61K 31/16, A61P 17/00

(54) **THERAPEUTIC CREAM FOR DERMATITIS**
THERAPEUTISCHE CREME GEGEN DERMATITIS
CREME THERAPEUTIQUE CONTRE LA DERMATITE

(43) Date of publication of application: 12.01.2005
(73) Proprietor: Santo, Tetsuo, Hamada-shi, Shimane 697-1322 (JP)
(72) Inventor: SANTO, Tetsuo, Hamada-shi, Shimane 697-1322 (JP); LI, Songhua, Izumo-shi, Shimane 693-0002 (JP); WANG, Ruwei, Lishushi, Zhejiangsheng 32300 (CN); IWASAKI, Sumio, Izumo-shi, Shimane 693-0003 (JP)
(74) Representative: Wittop Koning, Tom Hugo
(86) International application number: PCT/JP2002/003747
(87) International publication number: WO 2003/086433

(56) References cited:
- EP-A1- 0 308 210
- JP-A- 4 082 830
- JP-A- 4 356 423
- JP-A- 4 356 424
- JP-A- 5 043 449
- JP-A- 6 166 629
- JP-A- 6 211 673
- JP-A- 6 279 305
- JP-A- 7 138 173
- JP-A- 7 309 713
- JP-A- 8 040 922
- JP-A- 9 030 931
- JP-A- 9 087 189
- JP-A- 9 095 451
- JP-A- 9 124 500
- JP-A- 10 139 679
- JP-A- 63 022 506
- JP-A- 2000 044 481
- JP-A- 2000 103 718
- JP-A- 2000 169 383
- JP-A- 2000 239 178
- JP-A- 2001 226 213
- JP-A- 2002 047 193
- JP-A- 2002 053 485
- US-A- 5 449 519
- SHINGO YANO ET AL.: 'Antiallergic activity and of curcuma longa active principles and mode of action' JOURNAL OF TRADITIONAL MEDICINES vol. 12, no. 4, 1995, pages 269 - 272, XP002975329
- DINESH CHANDRA ET AL.: 'Anti-inflammatory and anti-arthritic activity of volatile oil of curcuma longa(Haldi)' INDIAN J. MED. RES. vol. 60, 1972, pages 138 - 142, XP000915712
- DIEL F. ET AL.: 'Tea of isatis tintoria (woad) responses by allergic patients in vivo and in vitro' AKTUELLE ERNAHRUNG KLINIK UND PRAXIS vol. 17, no. 1, 1992, pages 34 - 36, XP002975330
- YOSHIYUKI KIMURA ET AL.: 'Histamine-release effectors from angelica dahurica var. dahurica root' J. NAT. PROD. vol. 60, no. 3, 1997, pages 249 - 251, XP002975331
- LUGASI A. ET AL.: 'Flavonoid aglycons in foods of plant origin II. Fresh and fried fruits' ACTA ALIMENTARIA vol. 31, no. 1, 2002, pages 63 - 71, XP002968849

## Description

### TECHNICAL FIELD

The present invention relates to a cream for therapy of dermatitis, and relates to a cream for therapy of dermatitis, including eczema, which is suitable to treat various dermatitis, particularly, for example, atopic dermatitis, and in which extracts drawn from plants are used.

### BACKGROUND ART

Patients having dermatitis, particularly patients having atopic dermatitis, have suddenly increased in recent years. While the grounds for the sudden increase of patients having atopic dermatitis has yet not been sufficiently clarified, it is considered that the grounds are classified into three large groups. The first ground is a change in the eating habit. That is, by an increase in consumption of flesh and meat as well as dairy product such as butter, cheese and the like, changed from the conventional vegetable-centered diet, it is considered that the physical constitution itself has been changed. The second ground is a change in the living environment. That is, by a change from the conventional houses using wood, plaster, paper, rush-mat and the like to houses using various synthetic building materials, chemically synthesized size, chemical mat and the like, it is considered that various chemical substances contained in these building materials are released in the living environment resulting in the change in the physical constitution. In addition, irritation on the skin may be increased by a change from the conventional clothes made of natural material fibers such as wool, cotton and the like to clothes made of various chemical fibers resulting in increase of irritation onto the skin, and a change from washing with soaps to washing with synthetic detergents and dry-cleaning, and a use of shampoos, rinses, hair conditioners may also be grounds. As the third ground, it is considered that a level down in immunity is caused by speed-up in the living rhythm and raised level of work proceeded in all the aspect, resulting in exposure of infants and adults to excess stresses.

Atopic dermatitis is a disease occurred at from two- or three-months old to about ten years old when resistance power is poor and is known to be a disease accompanied by intense itch with wetting and erosion; the itch is characterized in that it gives a mental pain to the patients and aggravates symptoms by scratching; particularly, in the case of infant patients, it is painful not only for the patients themselves but also for parents and near relations.

Although various countermeasures have been examined and practiced for the prophylaxis or therapy of this atopic dermatitis, most of them are countermeasures belonging to the symptomatic therapy; particularly known are antihistaminic agents, antiallergic agents, antiphlogistic agents, steroidal agents and the like for the symptomatic therapy of western medicine, but all of them have been unsatisfactory in pharmacological effect and side effects.

For example, although antihistaminic agents and antiallergic agents have an action of suppressing itch, they have problems in the duration of effect and antiphlogistic effect, and are problematic in long-term administration for chronic itching because sometimes they bring about troubles in the daily life due to symptoms such as weariness, sleepiness and the like caused by administration.

Although steroidal agents have generally a high pharmacological effect, they are fundamentally drugs for suppressing symptoms, and sometimes the cure cannot be attained even by a long-term administration of steroidal agents; they are problematic because of their strong drug-characteristic side effects; for example, sometimes they cause dermatrophia in which the skin becomes thin like a flimsy, capillarectasia in which capillary blood vessel in the skin rises forming red-skin, and various infections such as fungal infection, folliculitis (pimple) , herpes and the like due to decrease in the immune power. Additionally, when a very large amount of a very strong steroidal agent is used within a short period, sometimes functional disorder of adrenal grand, shock and the like occurs. In another case, when use of steroidal agent is suddenly discontinued after a long-term use, problems arises that the daily life become difficult by a revival of symptoms suppressed before by the steroidal agent and a rebound phenomenon (jump back) in which symptoms such as itching, redness, swelling and the like increases more than before.

In addition, based on the fact that staphylococcus aureus and others were found in the diseased part of atopic dermatitis, application of Isodine, a disinfectant agent, has been practiced in some cases. Indeed the effect of application of Isodine has been confirmed in a skin on which bacteria is abundant, Isodine is effective only to bacteria floating on the surface of skin, and has no effect against bacteria within a biological membrane or bacteria invaded deeply in the skin. Not only that, Isodine is liable to cause a rash, and when once a rash is caused, the reaction is repeated and sometimes an ulcer is formed on the skin or a reaction such as shock or the like is caused. Moreover, sometimes hypothyroidism is induced.

Froma similar viewpoint against bacteria, sometimes super acidic water is used, but problems similar to those in Isodine arise.

In addition to the above-described countermeasures based on western medicine, treatments by Sino-Japanese medicament have also been practiced. For example, respective crude drug ingredients of Rhizoma coptidis detoxication soup and heat-clearing and wind-dispelling power as therapeutic agents for atopic dermatitis are Baikal skullcap, Coptis root, Gardenia fruit, Amur cork tree and Japanese Angelicae root, Chinese Fox-Glove root, gypsum, Saposhnikoviae radix, great Burdock achene, Akebiae stem, Anemarrhena rhizome, sesame, Cryptotympana atrata, lightyellow Sophora root; since they belong to anti-itching agents for suppressing itch or blood-activation agents for stopping pain by improving blood circulation and therefore respective drugs belong to the symptomatic therapy dealing with individual symptoms, these medicaments cannot be said to be medicaments for fundamental cure.

In addition, although drugs for pain such as ointments containing Sino-Japanese drugs have been prepared, these belong also to so-called symptomatic therapy and therefore are far from the fundamental cure by improvement of physical constitution.

Moreover, while the activity is mild, Sino-Japanese drugs for suppressing the aforementioned side effects have been proposed. For example, Japanese Patent Publication JP-A-6-166629 has proposed an agent for improving atopic dermatitis formed by mixing potent Bupleuri decoction and Angelica peony powder. Indeed side effects are suppressed in these agents for improving atopic dermatitis, there is a problem that its anti-itching effect and so on are not sufficient.

Beside, Japanese patent publication JP-A-8-301779 has proposed an external drug for atopic dermatitis containing as an active ingredient an extract solution from one, two or more plants selected from the group consisting of linden, lemonbalm, fenugreek, borage, Lingusticum chuanxiong Hort, pink pyrola, willowleaf swallowwort rhizome, Clerodendron cyrtophyllum, and Clinopodium chinense.

The Japanese patent document P2000044481 discloses a topical preparation for suppressing inflammation and itching due to atopic dermatitis and pruritus comprising a Sophora flavescens, Japanese Angelica root, and liquorice.

However, while the ointment for atopic dermatitis exerts some anti-itching effect and disease-improving effect by applying it onto the diseased part, there has been a problem that a sufficient effect was not obtained.

Therefore, the purpose of the present invention is to provide a cream for therapy of dermatitis which can treat atopic dermatitis or the like.

### DISCLOSURE OF THE INVENTION

The creams for therapy of dermatitis according to the present invention are as set forth in claims 1-4.

The families, main components and principal activities of the plants as raw materials for the respective extracts drawn from plants are described in the following:
(1) lightyellow Sophora root (Kuj in) (Sophora flavescens Ait.)
   Plant family: Leguminosae plant fravescens
   Main components: matorine, kurarinore
   Principal activities: antibacterial, antiviral, antiallergic
(2) Turmeric (Ukon) (Curcuma aromatica Salisb.)
   Plant family: Zingiberacea plant
   Main components: curcumol, curdion
   Principal activities: antibacterial, antiphlogistic,
   blood-circulation improving
(3) Magnolia bark (Kouboku) (Magnolia officinalis Rehd. et Wils.)
   Plant family: Magnoliaceae plant
   Main components: honoriol, β-eudesml
   Principal activities: antibacterial, antiphlogistic
(4) Moutan bark (botanpi) (Paeonia suffruticosa Andr.)
   Plant family: Paeoniaceae plant
   Main components: paeonol, benzoic acid, phytosterol
   Principal activities: antiphlogistic, blood-circulation improving
(5) Isatis leaf (Taiseiyou) (Isatis tinctoria L.)
   Plant family: Acantaceae plant
   Main components: indigo, indirubin, idican, trace element
   Principal activities: antibacterial, antiviral, antiallergic
   In addition, Isatis leafs prepared from Baphicacanthus cusia Bremek, Isatis indigotica fort, Polygonum tinctorium Ait., Clerodendron cyrtophyllum turcz. and the like can be used.
(6) Borneo camphor tree (Hyouhen) (Dryobalanops aromatica Gaertn.f.)
   Plant family: Diptercarpaceae plant. Crystals prepared by processing resin from Dipterocarpus retusus.
   Main components: volatile oil, α-borneol
   Principal activities: antibacterial, antiphlogistic, anti-itching

According to the above described cream for therapy of dermatitis, curing potential is elevated by antibacterial effect of Lightyellow Sophora root, turmeric, Magnolia bark, Isatis leaf, and Borneo camphor tree (Dryobalanops aromatica Gaertn. f.), antiviral and antiallergic effects of lightyellow Sophora root and Isatis leaf, antiphlogistic effect of turmeric, Magnolia bark, Moutan bark and Borneo camphor tree (Dryobalanops aromatica Gaertn. f.), blood-circulation improving effect of turmeric and Moutan bark, anti-itching effect of Borneo camphor tree (Dryobalanops aromatica Gaertn. f.), or by a synergistic effect of them, and also by a depression effect against allergens, and thus various dermatitis including atopic dermatitis can be treated.

The invention is also characterized in that an auxiliary agent is added to the above described extracts drawn from plants.

As the auxiliary agent, materials having various auxiliary activities are adopted, including one for assistance and enhancement of pharmacological effects, namely antibacterial, antiviral, antiallergic, antiphlogistic, blood-circulation and anti-itching effects, of aforementioned extracts drawn from plants, one for addition of pharmacological effects, for example, antifungal, sterilizing effects and others, which are lacking in aforementioned extracts drawn fromplants, one for improvement of easiness of applying as a cream in the form of an ointment by dissolving the aforementioned extracts drawn from plants, one for accelerating skin permeation so that the pharmacologically active ingredients applied onto the diseased part permeate deeply into inside of the skin, or further, one for accelerating keratinization of skin by the therapeutic effects of the pharmacologically active ingredients applied onto the diseased part, and so on.

According to the above cream for therapy of dermatitis, with the auxiliary agent, the therapeutic effect is promoted by assisting or enhancing the pharmacological effects of the extracts drawn from plants namely lightyellow Sophora root, turmeric, Magnolia bark, Moutan bark, Isatis leaf, and Borneo camphor tree (Dryobalanops aromatica Gaertn. f.), by adding a pharmacological effect lacking in the above described extracts drawn from plants, or by improving easiness of applying as a cream, or further, or by accelerating effect of skin permeation and acceleration effect of keratinization, and inconvenience such as side effects occurred by use continued for a period needed in therapy and the like can be avoided.

The invention is also characterized in that the auxiliary agent includes an auxiliary extract drawn from a plant, accelerator for skin permeation, accelerator for keratinization, mutton oil, alcohol and white soft paraffin.

According to the above cream for therapy of dermatitis, with the auxiliary extract drawn from a medicinal herb, the pharmacological effects of the extracts drawn fromplants namely lightyellow Sophora root, turmeric, Magnolia bark, Moutan bark, Isatis leaf, and Borneo camphor tree (Dryobalanops aromatica Gaertn. f.) can be assisted or enhanced, and the therapeutic effect can be promoted by adding a pharmacological effect as described above lacking in the above described extracts drawn from plants, by improving easiness of applying as a cream, or by accelerating agent for skin permeation acting for permeation of the pharmacologically active ingredients deeply under the skin of diseased part, and by accelerating agent for keratinization of skin acting for keratinization of the diseased part so that the metabolism in the diseased part is elevated.

Besides, mutton oil (purified lanolin) acts for adjustment of viscosity in the cream for therapy of dermatitis and is useful for holding of the pharmacologically active ingredients on the diseased part after evaporation of alcohol and water described below, alcohol acts for easiness of dissolving respective ingredients and is useful for imparting cold feeling on applying, and white soft paraffin is useful for easiness of applying onto the diseased part by keeping viscosity as a cream.

The invention is also characterized in that the above described auxiliary extract drawn from a plant contains extracts drawn from the group of Baikal skullcap, Amur cork tree, Angelicae Dahuricae root, lemon, smartweed and licorice.

The families, main components and principal activities of the above described auxiliary agent are described in the following:
(1) Baikal skullcap (Ougon) (Scutellaria baikcalensis Georgi.)
   Plant family: Labiatae plant
   Main component: baicalin, baicalein
   Principal activity: antibacterial, antiviral, antiallergic
(2) Amur cork tree (Oubaku) (Phellodendron amurense Rupr. )
   Plant family: Rutaceae plant
   Main components: berberine, phellodendorine
   Principal activity: antibacterial, antiphlogistic
(3) Angelicae Dahuricae root (Hakusi) (Angelica dahurica Benth. et Hook.)
   Plant family: Umbelliferae plant
   Main components: byak-anngelicin, imperatorin
   Principal activities: antibacterial, antiphlogistic, antiallergic
(4) Lemon (Lemon)
   Plant family: Rutaceae plant, food additive
   Main component: lemon acid
   Principal activities: antiallergic
(5) Smartweed (Kojou) (Polygonum cuspidatumSieb. et Zucc.)
   Plant family: Polygonaceae plant (Smartweed plant)
   Main components: glycosides, flabonoids
   Principal activities: antibacterial, antiviral, antiallergic
(6) Licorice (Kanzou) (Glycyrrhiza uralensis Fisch.)
   Plant family: Legminosae plant
   Main components: glycyrrheti acid, flabonoids
   Principal activities: antiallergic, antiphlogistic

According to the above cream for therapy of dermatitis, with the effects of respective auxiliary agents, the pharmacological effects, namely antibacterial, antiviral, antiallergic, antiphlogistic, blood-circulation improving, anti-itching effects and the like, of extracts drawn from plants consisting of lightyellow Sophora root, turmeric, Magnolia bark, Moutan bark, Isatis leaf and Borneo camphor tree (Dryobalanops aromatica Gaertn. f.) can be reinforced by antibacterial activity of Baikal skullcap, Amur cork tree, Angelicae Dahuricae root and smartweed, antiviral activity of Baikal skullcap and smartweed, antiallergic activity of Baikal skullcap, Angelicae Dahuricae root, lemon, smartweed and licorice and antiphlogistic activity of Amur cork tree, Angelicae Dahuricae root and licorice, resulting in a cream having a higher therapeutic effect.

The invention is also characterized in that the above described accelerating agent for skin permeation contains one, two or more selected from the group of Cnidii rhizoma, Japanese Angelicae root and dimethylsulfoxide.

The families, main components and principal activities of Cnidii rhizoma and Japanese Angelicae root, and properties and principal activities of dimethylsulfoxide are described in the following:
(1) Cnidii rhizoma (Senkyu) (Ligusticum chuanxiong Hort)
   Plant family: Umbelliferae plant
   Main components: volatile oil, tetramethylpyrazine, senkyunolide, ferulic acid
   Principal activities: skin permeation accelerating, blood-circulation accelerating, sedative, antispasmodic
(2) Japanese Angelicae root (Touki) (Angelica sinensis (olive) Diels.)
   Plant family: Umbelliferae plant
   Main components: volatile oil, ferulic acid, vitamin E, vitamin A, vitamin B₁₂
   Principal activities: skin permeation accelerating, blood-circulation accelerating, anemia improving, immunity regulating, antiallergic
(3) Dimethyl sulfoxide
   Properties: soluble in alcohol and water
   Principal activity: skin permeation accelerating

According to the above cream for therapy of dermatitis, the therapeutic effect can be elevated not only at the surface of skin but also in deep tissue of skin by permeating and penetrating effectively and deeply the pharmacologically active ingredients of the extracts drawn from plants into the subcutaneous tissue by the skin permeation accelerating action of Cnidii rhizoma, Japanese Angelicae root and dimethylsulfoxide, and moreover, the therapeutic effect can be elevated by blood-circulation accelerating, sedative and antispasmodic action of Cnidii rhizoma, as well as blood-circulation accelerating, anemia improving, immunity regulating and antiallergic action of Japanese Angelicae root.

The invention is also characterized in that the above described accelerating agent for keratinization contains salicylic acid and resorcinol.

The properties and principal activities of salicylic acid and resorcinol are described briefly in the following:
(1) Salicylic acid (Acidum salicylicum) Properties: easily soluble in alcohol, soluble in water Principal activities: keratinization accelerating, antiseptic
(2) Resorcinol
   Properties: soluble in alcohol and water
   Principal activities: keratinization accelerating, antiseptic, antipruritic, antimold

According to the above described cream for therapy of dermatitis, the therapeutic effect can be increased by accelerating keratinization of already cured old tissue by keratinization accelerating action of salicylic acid and/or resorcinol resulting in dropping out from the diseased part thereby rising new tissue up to the skin surface, together with a therapeutic effect given by the therapeutically active ingredients in the extracts drawn from plants and auxiliary agents onto the diseased part.

When the extracts drawn from plants is less than 53%, appearance of medical effect is slow or no improving effect appears; when it exceeds 89%, there is a fear that a side effect in which skin become red and swelled or the symptom become worse occurs. When accelerating agent for skin permeation is less than 8%, appearance of medical effect is slow or no improving effect appears; when it exceeds 10%, there is a fear that a side effect in which skin become red and swelled or the symptom become worse occurs. When accelerating agent for keratinization is less than 7%, appearance of medical effect is slow or no improving effect appears; when it exceeds 10%, there is a fear that a side effect in which skin become red and swelled or the symptom become worse occurs.

According to the above described cream for therapy of dermatitis, the respective auxiliary agents are contained with a good balance, not only the therapy of surface layer in the diseased part obtainable by a high pharmacological effects and permeation effect of the pharmacologically active ingredients into a deep subcutaneous tissue by the skin permeation accelerating agent, but also a high therapeutic effects of dermatitis is obtainable by a therapeutic action from the deep subcutaneous tissue and accelerating effect for keratinization of already cured old tissue by keratini zation accelerating agent, and a cream having a suppressed excess irritation by applying can be obtained.

The invention is characterized in that the volume ratios of respective ingredients are lightyellow Sophora root, 2.7 to 3.3%; turmeric, 1.8 to 2.2%; Magnolia bark, 1.8 to 2.2%; Moutan bark, 1.8 to 2.2%; Isatis leaf, 0.9 to 1.1%; Borneo camphor tree (Dryobalanops aromatica Gaertn. f.), 0.9 to 1.1%; Baikal skullcap, 1.8 to 2.2%; Amur cork tree, 1.8 to 2.2%; Angelicae Dahuricae root, 0.9 to 1.1%; lemon, 0 to 3%; smartweed, 0 to 1.1%; licorice, 0 to 0.55%; Cnidii rhizoma, 0.45 to 1.1%; Japanese Angelica root, 0 to 0.55%; salicylic acid, 0.45 to 0.55%; resorcinol, 0.45 to 0.55%; mutton oil, 2.7 to 3.3%; alcohol, 2.7 to 3.3%; and white soft paraffin, 63 to 78% (claim 1).

When respective ingredients other than mutton oil, alcohol and white soft paraffin among the above described ingredients are less than the lower limit of the above range, appearance of medical effect is slow or no medical effect appears; when it exceeds the upper limit of the above range, there is a fear that a side effect in which skin become red and swelled or the symptom become worse occurs.

According to the above described cream for therapy of dermatitis, a high therapeutic effect against atopic dermatitis can be obtained owing to the fact that well-balanced antibacterial, antiviral, antiallergic, antiphlogistic, blood-circulation accelerating and anti-itching effects of lightyellow Sophora root, turmeric, Magnolia bark, Moutan bark, Isatis leaf and Borneo camphor tree (Dryobalanops aromatica Gaertn. f.) as the main extracts drawn from plants, assisting action and enhancing action for pharmacological activities by Baikal skullcap, Amur Cork Tree, Angelicae Dahuricae Root, lemon, smartweed, licorice as the auxiliary extracts drawn from plants, skin permeation accelerating action of Cnidii rhizoma and/or Japanese Angelicae root, and keratinization accelerating action of salicylic acid and/or resorcinol are obtained.

The invention is also characterized in that the volume ratios of respective ingredients are Lightyellow Sophora root, 3%; turmeric, 2%; Magnolia bark, 2%; Moutan bark, 2%; Isatis leaf, 1%; Borneo camphor tree (Dryobalanops aromatica Gaertn. f.), 1%; Baikal skullcap, 2%; Amur cork tree, 2%; Angelicae Dahuricae root, 1%; lemon, 3%; smartweed, 1%; licorice, 0.5%; Cnidii rhizoma, 0.5%; Japanese Angelica root, 0.5%; salicylic acid, 0.5%; resorcinol, 0.5%; mutton oil, 3%; alcohol, 3%; and white soft paraffin, 70.5% (claim 2).

According to the above described cream for therapy of dermatitis, the highest therapeutic effect against atopic dermatitis can be obtained owing to the fact that very well-balanced antibacterial, antiviral, antiallergic, antiphlogistic, blood-circulation accelerating and anti-itching effects of lightyellow Sophora root, turmeric, Magnolia bark, Moutan bark, Isatis leaf and Borneo camphor tree (Dryobalanops aromatica Gaertn. f.) as the main extracts drawn from plants, assisting action and enhancing action for pharmacological activities by Baikal skullcap, Amur cork tree, Angelicae Dahuricae root, lemon, smartweed, licorice as the auxiliary extracts drawn from plants, skin permeating action of Cnidii rhizoma and/or Japanese Angelicae root, and keratinization accelerating action of salicylic acid and/or resorcinol are obtained.

The invention is also characterized in that the volume ratios of respective ingredients are light yellow Sophora root, 2.7 to 3.3%; turmeric, 1.8 to 2.2%; Magnolia bark, 1.8 to 2.2%; Moutan bark, 1.8 to 2.2%; Isatis leaf, 0.9 to 1.1%; Borneo camphor tree (Dryobalanops aromatica Gaertn. f.), 0.9 to 1.1%; Baikal skullcap, 1.8 to 2.2%; Amur cork tree, 1.8 to 2.2%; Angelicae Dahuricae root, 0.9 to 1.1%; lemon, 0 to 3%; smartweed, 0 to 1.1%; licorice, 0 to 0.55%; dimethylsulfoxide, 4.5 to 5.5%; salicylic acid, 0.45 to 0.55%; resorcinol, 0.45 to 0.55%; mutton oil, 2.7 to 3.3%; alcohol, 2.7 to 3.3%; and white soft paraffin, 60 to 73% (claim 3).

According to the above described cream for therapy of dermatitis, a high therapeutic effect against atopic dermatitis can be obtained owing to the fact that well-balanced antibacterial, antiviral, antiallergic, antiphlogistic, blood-circulation accelerating and anti-itching effects of lightyellow Sophora root, turmeric, Magnolia bark, Moutan bark, Isatis leaf and Borneo camphor tree (Dryobalanops aromatica Gaertn. f.) as the main extracts drawn from plants, assisting action and enhancing action for pharmacological activities by Amur cork tree, Angelicae Dahuricae root, lemon, smartweed, licorice as the auxiliary extracts drawn from plants, skin permeation accelerating action of dimethylsulfoxide, and keratinization accelerating action of salicylic acid and/or resorcinol are obtained.

The invention is also characterized in that the volume ratios of respective ingredients are lightyellow Sophora root, 3%; turmeric, 2%; Magnolia bark, 2%; Moutan bark, 2%; Isatis leaf, 1%; Borneo camphor tree (Dryobalanops aromatica Gaertn. f.), 1%; Baikal skullcap, 2%; Amur cork tree, 2%; Angelicae Dahuricae root, 1%; lemon, 3%; smartweed, 1%; licorice, 0.5%; dimethylsulfoxide, 5%; salicylic acid, 0.5%; resorcinol, 0.5%; mutton oil, 3%; alcohol, 3%; and white soft paraffin, 66.5% (claim 4).

According to the above described cream for therapy of dermatitis, the highest therapeutic effect against atopic dermatitis can be obtained owing to the fact that very well-balanced antibacterial, antiviral, antiallergic, antiphlogistic, blood-circulation accelerating and anti-itching effects of lightyellow Sophora root, turmeric, Magnolia bark, Moutan bark, Isatis leaf and Borneo camphor tree (Dryobalanops aromatica Gaertn. f.) as the main extracts drawn from plants, assisting action and enhancing action for pharmacological activities by Amur cork tree, Angelicae Dahuricae root, lemon, smartweed, licorice as the auxiliary extracts drawn from plants, skin permeation accelerating action of dimethylsulfoxide, and keratinization accelerating action of salicylic acid and/or resorcinol are obtained.

### BRIF DESCRIPTION OF DRAWINGS

[FIG. 1]
   (A) is a photograph showing the hollow of right hand when the patient in CASE 1 relating to the cream according to the invention has visited a doctor for initial consultation.
   (B) is a photograph showing the hollow of right hand after treatment.
[FIG. 2]
   (A) is a photograph showing the sole of left foot when the patient in CASE 1 relating to the cream according to the invention has visited a doctor for initial consultation.
   (B) is a photograph showing the sole of left foot after treatment.
[FIG. 3]
   (A) is a photograph showing the lower abdominal region when the patient in CASE 2 relating to the cream according to the invention has visited a doctor for initial consultation.
   (B) is a photograph showing the lower abdominal region after treatment.
[FIG. 4]
   (A) is a photograph showing the face when the patient in CASE 3 relating to the cream according to the invention has visited a doctor for initial consultation.
   (B) is a photograph showing the face after treatment.
[FIG. 5]
   (A) is a photograph showing the face when the patient in CASE 4 relating to the cream according to the invention has visited a doctor for initial consultation.
   (B) is a photograph showing the face after treatment.
[FIG. 6]
   (A) is a photograph showing the backside of right hand when the patient in CASE 4 relating to the cream according to the invention has visited a doctor for initial consultation.
   (B) is a photograph showing the backside of right hand after treatment.
[FIG. 7]
   (A) is a photograph showing the face when the patient in CASE 5 relating to the cream according to the invention has visited a doctor for initial consultation.
   (B) is a photograph showing the face after treatment.
[FIG. 8]
   (A) is a photograph showing the fingers of right hand when the patient in CASE 6 relating to the cream according to the invention has visited a doctor for initial consultation.
   (B) is a photograph showing the fingers of right hand after treatment.
[FIG. 9]
   (A) is a photograph showing the sole of right foot when the patient in CASE 7 relating to the cream according to the invention has visited a doctor for initial consultation.
   (B) is a diagram showing the sole of right foot after treatment.
[FIG. 10]
   (A) is a photograph showing the face when the patient in CASE 8 relating to the cream according to the invention has visited a doctor for initial consultation.
   (B) is a photograph showing the face after treatment.

### BEST MODE FOR CARRYING OUT THE INVENTION

The respective ingredients and weight ratios in the cream for therapy of dermatitis (atopic cream) A:
lightyellow Sophora root, 3%; turmeric, 2%; Magnolia bark, 2%; Moutan bark, 2%; Isatis leaf, 1%; Borneo camphor tree (Dryobalanops aromatica Gaertn. f.), 1%; Baikal Skullcap, 2%; Amur cork tree, 2%; Angelicae Dahuricae root, 1%; lemon, 3%; smartweed, 2%; licorice, 0.5%; Cnidii rhizoma, 0.5%; Japanese Angelica root, 0.5%; salicylic acid, 0.5%; resorcinol, 0.5%; mutton oil, 3%; alcohol, 3%; white soft paraffin, 70.5%.

The respective ingredients and weight ratios in the cream for therapy of dermatitis (atopic cream) B:
lightyellow Sophora root, 3%; turmeric, 2%; Magnolia bark, 2%; Moutan bark, 2%; Isatis leaf, 1%; Borneo camphor tree (Dryobalanops aromatica Gaertn. f.), 1%; Baikal skullcap, 2%; Amur cork tree, 2%; Angelicae Dahuricae root, 1%; lemon, 3%; smartweed, 1%; licorice, 0.5%; dimethylsulfoxide, 5%; salicylic acid, 0.5%; resorcinol, 0.5%; mutton oil, 3%; alcohol, 3%; white soft paraffin, 66.5%.

A significant therapeutic effect can be obtained by application of the cream (A, B) alone for therapy of dermatitis onto the diseased part; however, a more significant effect could be obtained by concomitant use of drinking of a tea for therapy of dermatitis (atopic tea), described below, and applying of a lotion (atopic lotion) (A, B) for therapy of dermatitis, described below, all being developed by the applicant, onto the diseased part. In the following, the 3 times a day applying of the atopic cream (A, B) and the atopic lotion (A, B) and the drinking of 3 g of the atopic tea were respectively carried out at morning, noon and night.

The respective ingredients and weight ratios in the tea for therapy of dermatitis (atopic tea):
The weights of extract ingredients drawn from respective medical herbs per g of a drinkable tea in the form of powders or granules are: lightyellow Sophora root, 0.1 g; Isatis leaf, 0.1 g; Terminalia fruit, 0.02 g; Japanese Angelica root, 0.05 g; Oldenlandia diffusa, 0.1 g; Smilax glabra, 0.12 g; Dried tangering peel, 0.05 g; chrysanthenum flower, 0-1 g; corydalis, 0.02 g; peppermint, 0.01 g; Baikal skullcap, 0.05 g; Lithospermum, 0.1 g; Kudingcha, 0.05 g; smartweed, 0.1 g; and licorice, 0.03 g.

The respective ingredients and volume ratios of lotion for therapy of dermatitis (atopic lotion) A:
lightyellow Sophora root, 3%; turmeric, 2%; Magnolia bark, 2%; Moutan bark, 2%; Isatis leaf, 1%; Borneo camphor tree (Dryobalanops aromatica Gaertn. f.), 1%; Amur cork tree, 2%; Angelicae Dahuricae root, 1%; lemon, 3%; smartweed, 2%; licorice, 0.5%; Cnidii rhizoma, 0.5%; Japanese Angelica root, 0.5%; salicylic acid, 0.5%; resorcinol, 0.5%; alcohol, 30%; water, 48.5%.

The respective ingredients and volume ratios of lotion for therapy of dermatitis (atopic lotion) B:
lightyellow Sophora root, 3%; turmeric, 2%; Magnolia bark, 2%; Moutan bark, 2%; Isatis leaf, 1%; Borneo camphor tree (Dryobalanops aromatica Gaertn. f.), 1%; Amur cork tree, 2%; Angelicae Dahuricae root, 1% ; lemon, 3%; smartweed, 2%; licorice, 0. 5%; dimethylsulfoxide, 5%; salicylic acid, 0.5%; resorcinol, 0.5%; alcohol, 26%; water, 48.5%.

### [CASE 1]

| | |
|---|---|
| Patient | Distinction of sex: female |
| | Date of birth: April 23, 1994 (Heisei 6) |
| | Age at initial consultation: 7 years old |

Initial consultation: June 14, 2001 (Heisei 13)
Medical history: two years ago, eczema occurred in hands and legs; thereafter, cracked skin occurred. No improvement in symptom was observed by use of steroidal agent and others. Physical examination: hands and legs are severely keratinized and cracked; dry and rough.
Prescription: External: application of 3 times a day of the atopic cream A on the diseased part.
Consequence: after 3 weeks, eczema on both hands and legs commenced.

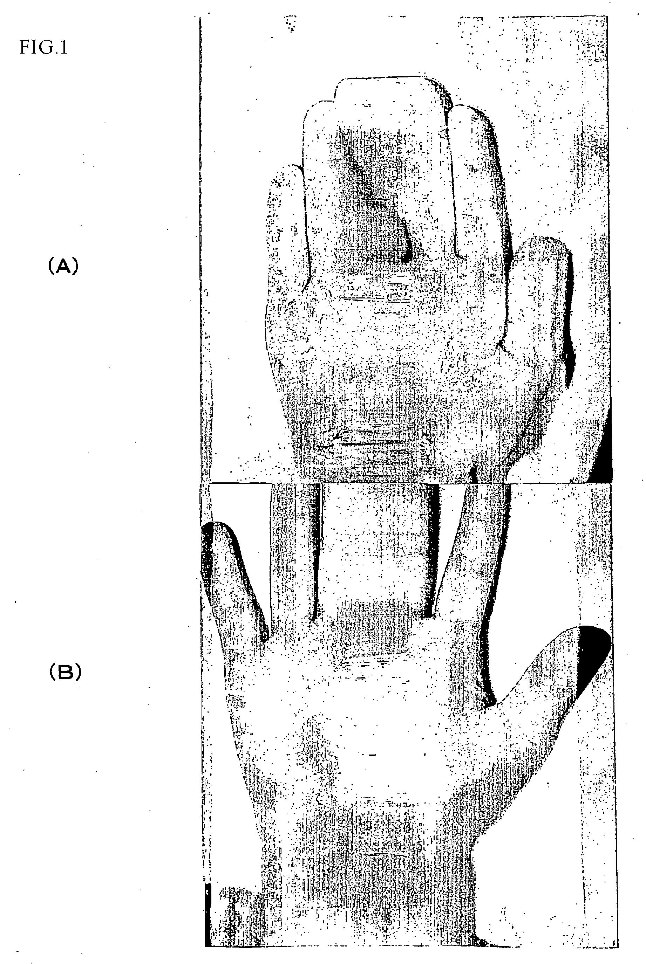

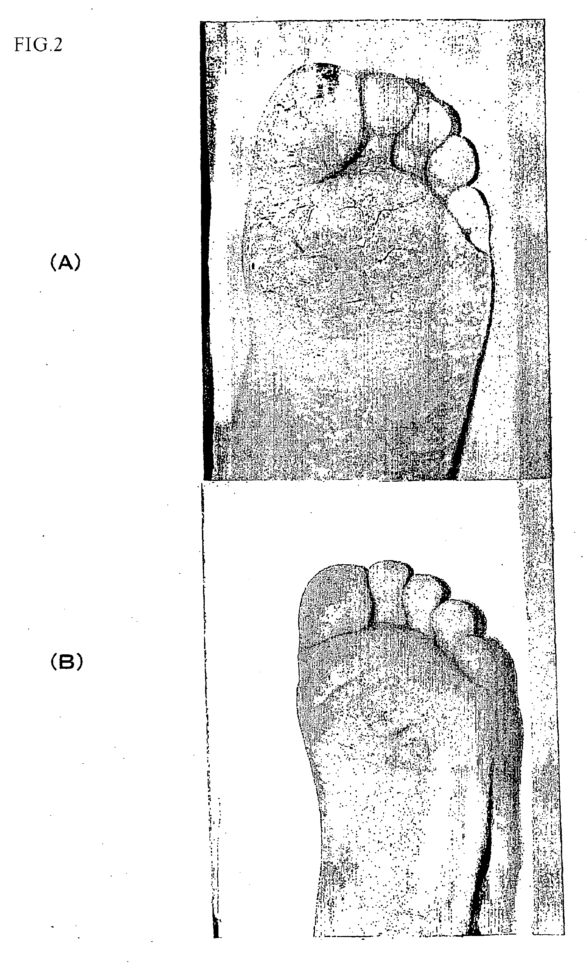

FIG. 1 (A) shows the condition of the hollow of right hand at the initial consultation; FIG. 1 (B) shows the condition of the hollow of right hand after 3 weeks of treatment. FIG. 2 (A) shows the condition of the sole of left foot at the initial consultation. FIG. 2 (B) shows the condition of the sole of left foot after 3 weeks of treatment.

### [CASE 2]

| | |
|---|---|
| Patient | Distinction of sex: female |
| | Date of birth: July 9, 1996 (Heisei 8) |
| | Age at initial consultation: 3 years old |

Initial consultation: March 29, 1999 (Heisei 11)
Medical history: onset was from 1 year after birth. Physical examination: particularly severe dermatitis on a part contacting with a diaper; the part is reddish and swollen, dry and rough.
Prescription: external application of 3 times a day of the atopic cream B on the diseased part
Consequence: effect was gradually appeared from 1 month; now, after 2 months, redness and swelling was improved. Afterpassage of 2 months, drinking of atopic tea was continued.

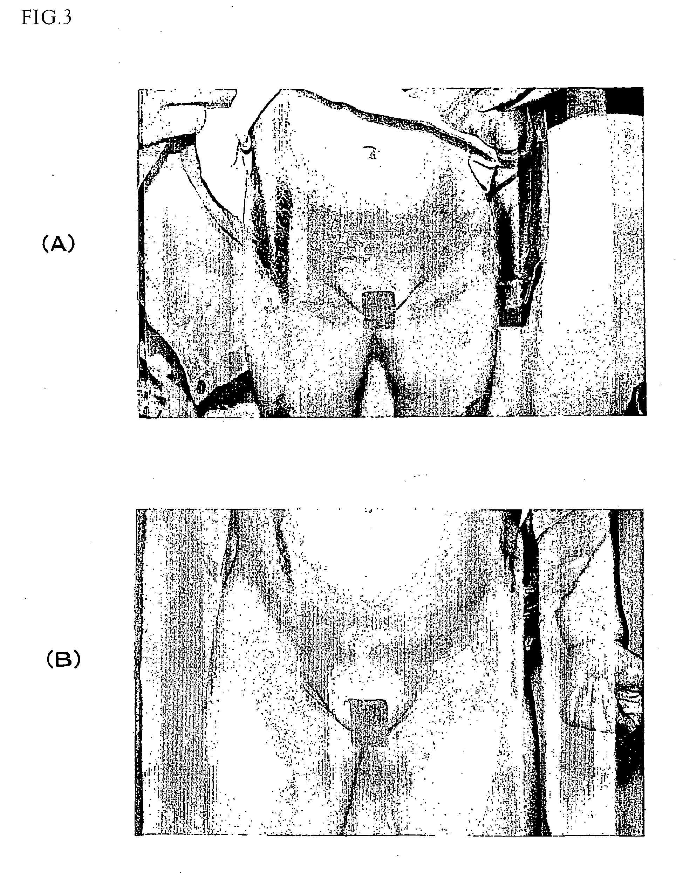

FIG. 3 (A) shows the condition of the lower abdominal region at the initial consultation. FIG. 3 (B) shows the condition of the lower abdominal region after treatment of 2 months.

### [CASE 3]

| | |
|---|---|
| Patient | Distinction of sex: female |
| | Date of birth: October 30, 1986 (Showa 61) |
| | Age at initial consultation: 12 years old |

Initial consultation: August 31, 1998 (Heisei 10)
Medical history: onset of atopy was after birth and accompanied by asthma; Gradual aggravation from elementally school girl. No improvement was observed after use of steroidal agent.
Physical examination: face is reddish and swollen with significant exfoliation of skin; skin of total body is rough with eczema and burn. Lichenification was found locally and there is a strong itching.
Prescription: external: application of 3 times a day of atopic cream A and atopic lotion A on the diseased part.
Consequence: after 3 months, the symptom was significantly improved. Afterwards, only the drinking of the atopic tea was continued, and the course is in good order.

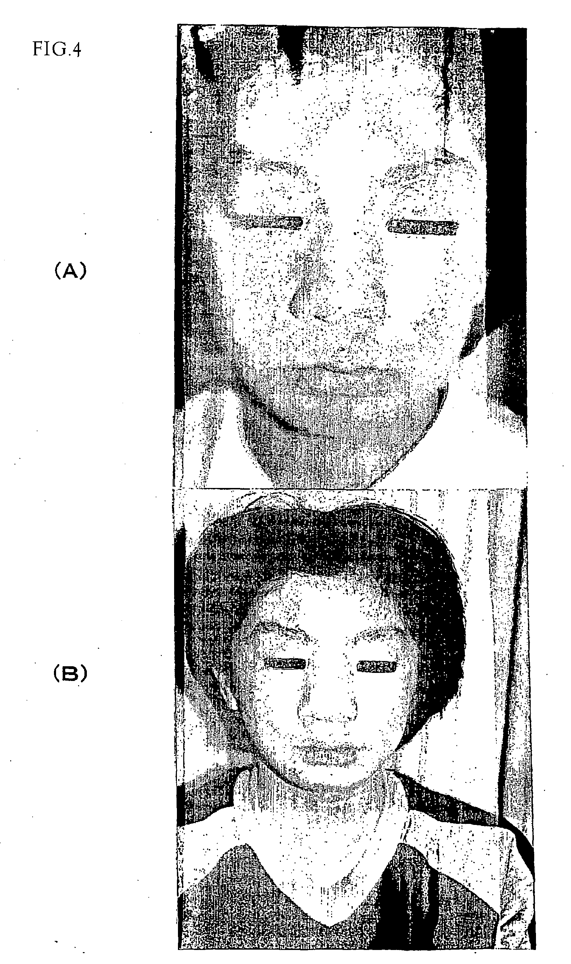

FIG. 4 (A) shows the condition of the face at the initial consultation; FIG. 4 (B) shows the condition of the face when 3 months were passed after starting the treatment.

### [CASE 4]

| | |
|---|---|
| Patient | Distinction of sex: female |
| | Date of birth: January 7, 1975 (Showa 50) |
| | Age at initial consultation: 23 years old |

Initial consultation: March 8, 2001 (Heisei 13)
Medical history: onset of atopy was at about elementary school girl age. No improvement was observed on use of steroidal agent. Two years ago, aggravation was found after delivery; exanthema and flare were found on all of face, extremities and trunk.
Physical examination: exanthema, flare, regional erosion, strong itch and burn were found on face.
Skin of both hands is reddish, tumefacient and cracked. Lichenification was found locally.
Prescription: external: application of 3 times a day of atopic cream A and atopic lotion A on the diseased part.
Internal: drinking of 3 g per day of the atopic tea.
Consequence: after 3 weeks, eczema, flare, erosion, cracking and so on were almost completely cured; significant improvement of itching was found.

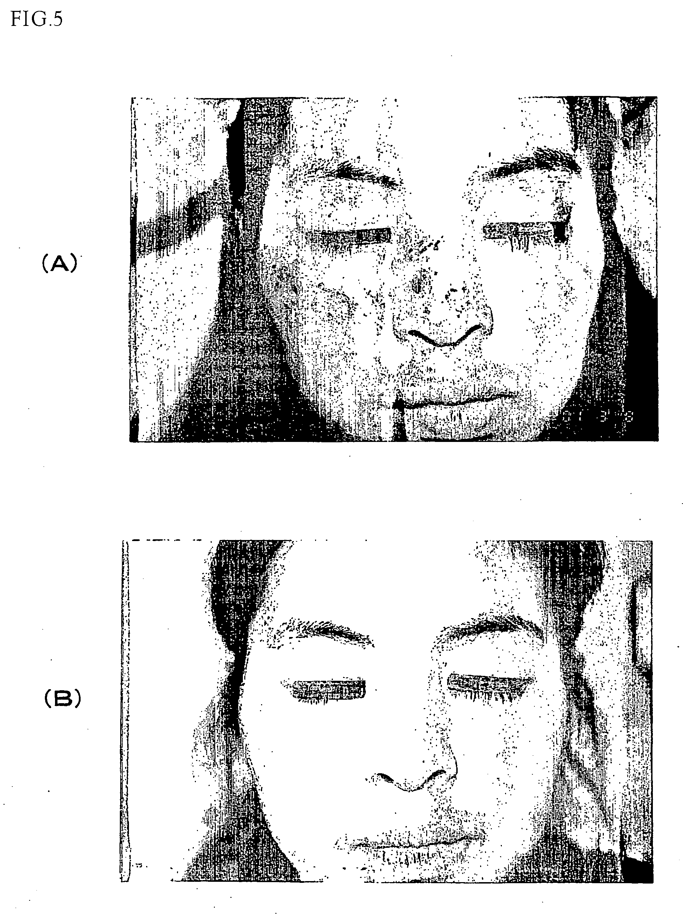

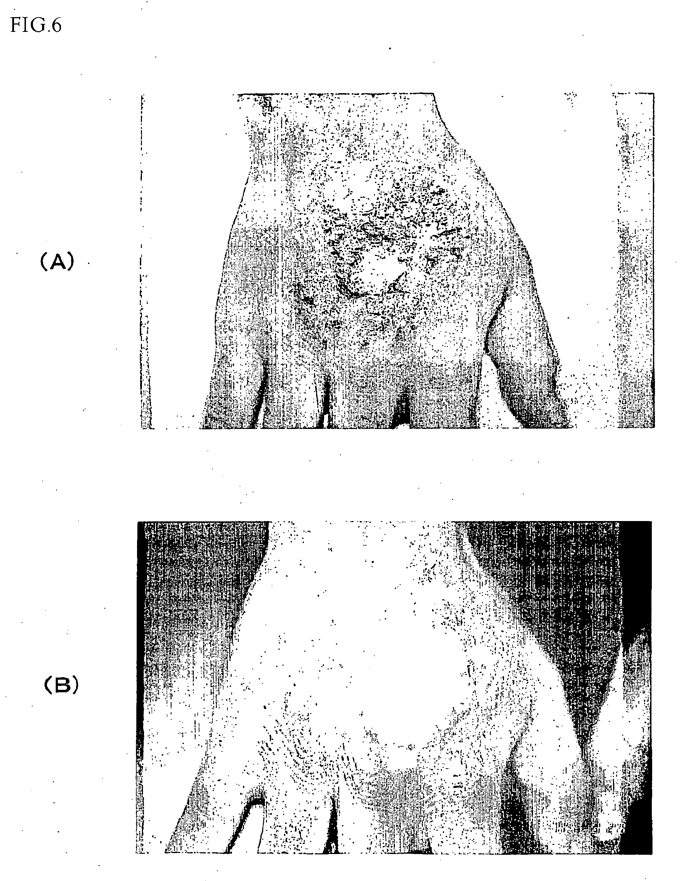

FIG. 5 (A) shows the condition of the face at the initial consultation; FIG. 5 (B) shows the condition of the face on day 13 after the treatment. FIG. 6 (A) shows the condition of the backside of right hand at the initial consultation; FIG 6 (B) shows the condition of the backside of right hand on day 20 after the treatment.

### [CASE 5]

| | |
|---|---|
| Patient | Distinction of sex: male |
| | Date of birth: March 15, 1999 (Heisei 11) |
| | Age at initial consultation: 0 year old (24 days after birth) |

Initial consultation: April 8, 1999 (Heisei 11)
Medical history: onset of eczema was 2 weeks after birth on face as main part, head, neck and round, ears, trunk and others. No improvement was found after use of commercially available drug.
Physical examination: eczema, exanthema and swelling on face, head, neck and round; regional pus.
Prescription: External: application of 3 times a day of atopic cream B and atopic lotion B on the diseased part.
Internal: Drinking of 3 g per day of the atopic tea.
Consequence: after 2 weeks, the skin manifestation was significantly improved, and 2 months later, the symptoms were settled and the treatment was discontinued.

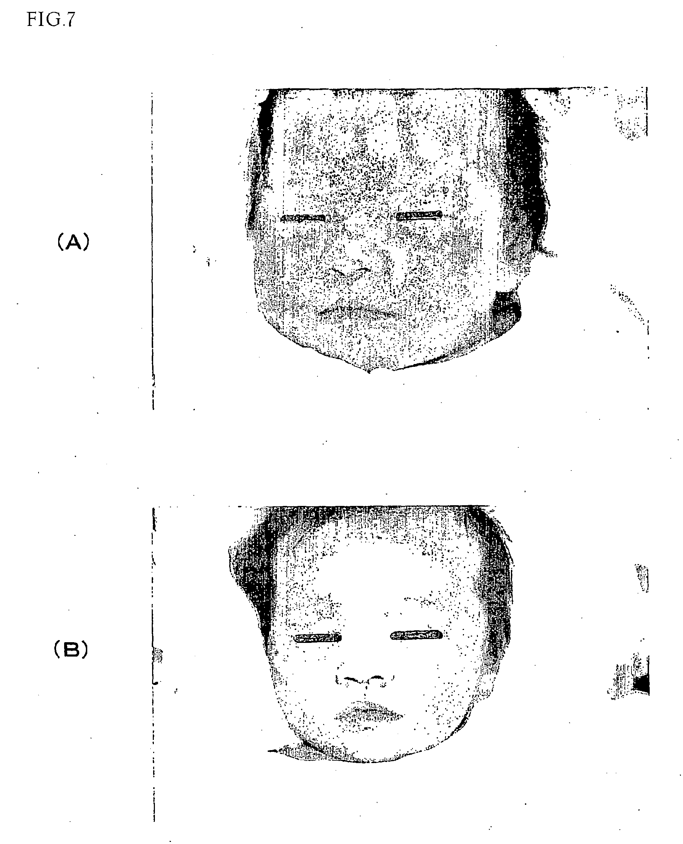

FIG. 7 (A) shows the condition of the face at the initial consultation; FIG. 7 (B) shows the condition of the face after 2 months.

### [CASE 6]

| | |
|---|---|
| Patient | Distinction of sex: male |
| | Date of birth: January 21, 1976 (Showa 51) |
| | Age at initial consultation: 22 years old |

Initial consultation: December 11, 1998 (Heisei 10)
Medical history: onset of atopy was at middle school boy age; gradual aggravation occurred during university student age; no improvement was observed by use of steroidal agent.
Physical examination: flare and eczema on face and head; dry and rough. Severe eczema on both hands; strong erosion, pus, itching and burn. Prescription: external: application of 3 times a day of atopic cream B and atopic lotion B on the diseased part.
Internal: drinking of 3 g per day of the atopic tea.
Consequence: after 3 weeks, eczema on both hands was almost completely cured.
Improving effect was observed in symptoms on face and head.

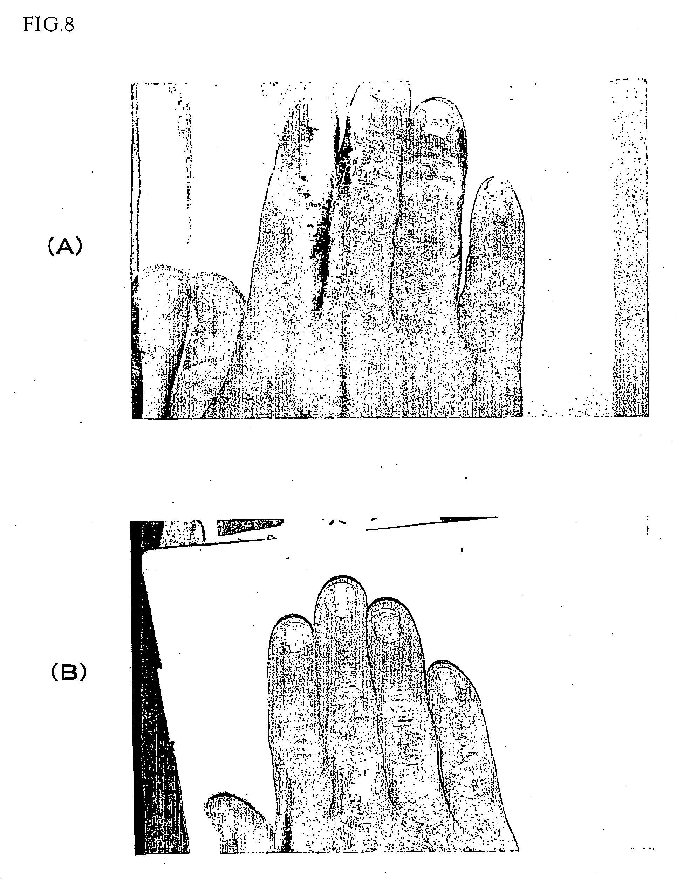

FIG. 8 (A) shows the condition of the fingers of right hand at the initial consultation; FIG. 8 (B) shows the condition of the fingers of right hand after 3 weeks.

### [CASE 7]

| | |
|---|---|
| Patient | Distinction of sex: female |
| | Date of birth: June 12, 1984 (Showa 59) |
| | Age at initial consultation: 14 years old |

Initial consultation: December 20, 1998 (Heisei 10)
Medical history: onset of atopy was at infancy. One year ago, aggravation occurred and onset was found on all of face, trunk and extremities; erosion and pus were found. Particularly, exanthema on gluteal region and both lower limb was severe and there is a strong itching and burn.
Crack on the sole of right foot was found.
Prescription: external: application of 3 times a day of atopic cream B and atopic lotion A on the diseased part.
Internal: drinking of 3 g per day of the atopic tea.
Consequence: after 2 weeks, improvement of symptoms was found. Crack on the sole of right foot was completely cured. Afterwards, only the drinking of the cream was continued, and the course is in good order.

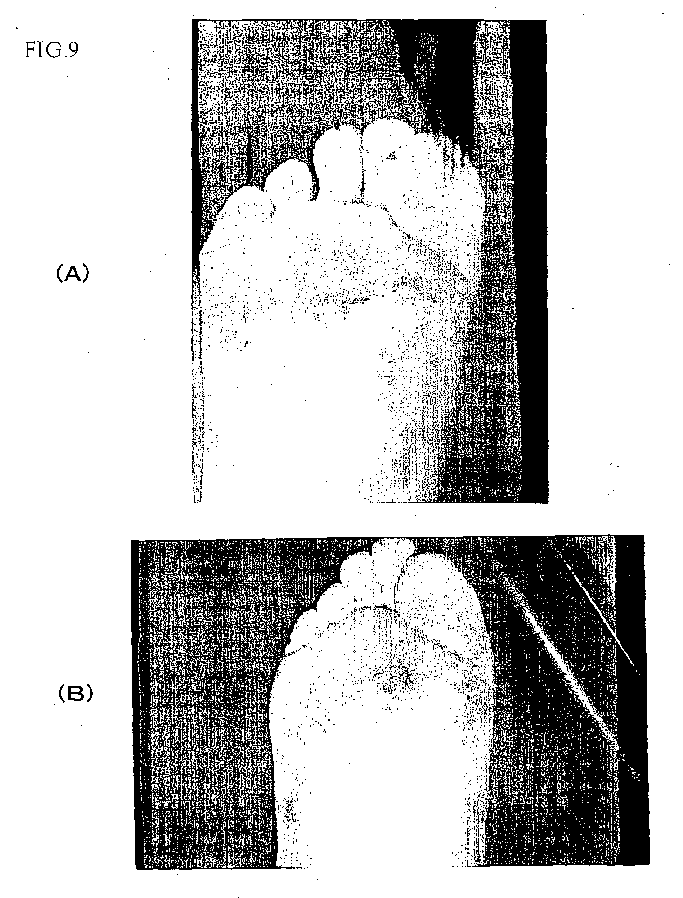

FIG 9 (A) shows the condition of the sole of right foot at the initial consultation; FIG 9 (B) shows the condition of the sole of right foot after 2 weeks.

### [CASE 8]

| | |
|---|---|
| Patient | Distinction of sex: male |
| | Date of birth: December 11, 1980 (Showa 55) |
| | Age at initial consultation: 20 years old |

Initial consultation: April 1, 1999 (Heisei 11)
Medical history: onset of atopy was after birth; steroid therapy was attempted at elementary to middle school boy age but no effect was found and the therapy was discontinued. Thereafter, antihistaminic agent alone was taken.
Physical examination: exanthema, dark redness and pigmentation on face; red swelling on neck, trunk and joints in extremities; many exfoliation; lichenification was found locally; itching is strong.
Prescription: external: application of 3 times a day of atopic cream A and atopic lotion B on the diseased part.
Internal: drinking of 3 g per day of the atopic tea.
Consequence: after 2 months, eczema, dark redness and pigmentation on face were almost completely cured. Eczema on trunk and extremities were significantly improved and the course afterwards is in good order.

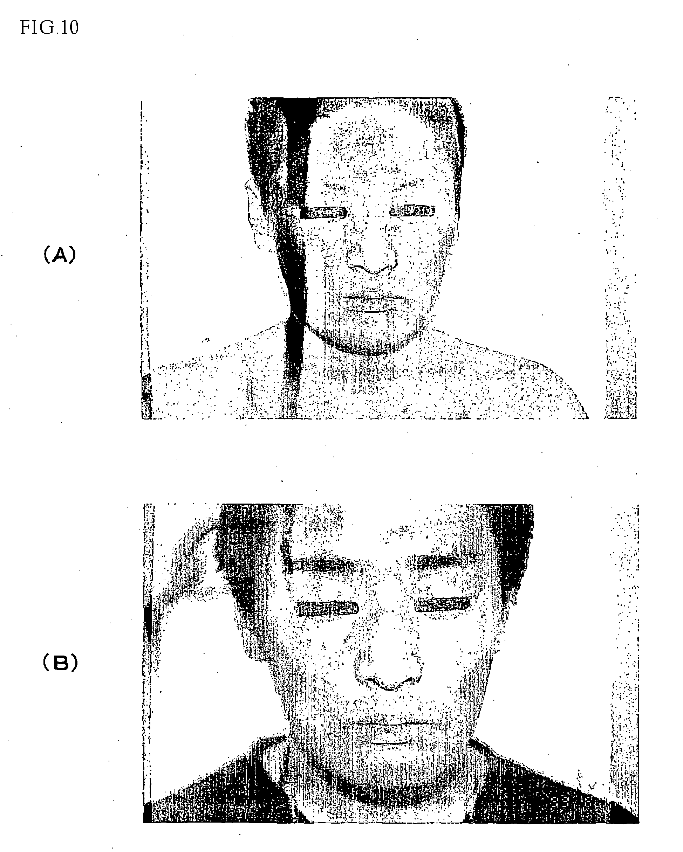

FIG. 10 (A) shows the condition of the face at the initial consultation; FIG 10 (B) shows the condition of the face after 2 months.

As shown in CASE 1 and CASE 2, an excellent therapeutic effect can be obtained by only applying of the cream for therapy of dermatitis; a more significant therapeutic effects can be obtained by concurrently occurred effect of the improvement of physical constitution from the inside of the body and direct therapeutic effect to the diseased part when an atopic tea is taken or an atopic cream (A, B) is applied concomitantly in the initial stage of the therapy or during the total period of the therapy.

Besides, although description in the above examples relates to cases wherein the accelerating agent for skin permeation consisting of Cnidii rhizoma plus Japanese Angelicae root was used in the cream A for therapy of dermatitis and cases wherein the accelerating agent for skin permeation consisting of dimethylsulfoxide was used in the cream B for therapy of dermatitis, similarly significant effects could be obtained in cases wherein the accelerating agent for skin permeation consisting of Cnidii rhizoma or Japanese Angelicae root alone was used, cases wherein the accelerating agent for skin permeation consisting of Cnidii rhizoma plus dimethylsulfoxide was used, cases wherein the accelerating agent for skin permeation consisting of Japanese Angelicae root plus dimethylsulfoxide was used, and cases wherein the accelerating agent for skin permeation consisting of Cnidii rhizoma plus Japanese Angelicae root plus dimethylsulfoxide was used.

## Claims

1. A cream for therapy of dermatitis **characterized in that** the volume ratios of respective ingredients are lightyellow Sophora root, 2.7 to 3.3%; turmeric, 1.8 to 2.2%; Magnolia bark, 1.8 to 2.2%; Moutan bark, 1.8 to 2.2%; Isatis leaf, 0.9 to 1.1%; Borneo camphor tree (Dryobalanops aromatica Gaertn. f.), 0.9 to 1.1%; Baikal skullcap, 1.8 to 2.2%; Amur cork tree, 1.8 to 2.2%; Angelicae Dahuricae root, 0.9 to 1.1 lemon, 0 to 3%; smartweed, 0 to 1.1%; licorice, 0 to 0.55%; Cnidii rhizoma, 0.45 to 1.1%; Japanese Angelica root, 0 to 0.55%; salicylic acid, 0.45 to 0.55%; resorcinol, 0.45 to 0.55%; mutton oil, 2.7 to 3.3%; alcohol, 2.7 to 3.3%; and white soft paraffin, 63 to 78%.

2. A cream for therapy of dermatitis **characterized in that** the volume ratios of respective ingredients are lightyellow Sophora root 3%; turmeric, 2%; Magnolia bark, 2%; Moutan bark, 2%; Isatis leaf, 1%: Borneo camphor tree (Dryobalanops aromatica Gaertn. f.), 1%; Baikal skullcap, 2%; Amur cork tree, 2%; Angelicae Dahuricae root, 1%; lemon, 3%; smartweed, 1%, licorice, 0.5%: Cnidii rhizoma, 0.5%; Japanese Angelica root, 0.5%; salicylic acid, 0.5%; resorcinol, 0.5%; mutton oil, 3%; alcohol, 3%; and white soft paraffin. 70.5%.

3. A cream for therapy of dermatitis **characterized in that** the volume ratios of respective ingredients are lightyellow Sophora root, 2.7 to 3.3%; turmeric, 1.8 to 2.2%; Magnolia bark, 1.8 to 2.2%; Moutan bark, 1.8 to 2.2%; Isatis leaf, 0.9 to 1.1%; Borneo camphor tree (Dryobalanops aromatica Gaertn. f.), 0.9 to 1.1%; Baikal skullcap 1.8 to 2.2%; Amur cork tree, 1.8 to 2.2%; Angelicae Dahuricae root, 0.9 to 1.1% lemon, 0 to 3% smartweed, 0 to 1.1%; licorice, 0 to 0.55%; dimethylsulfoxide, 4.5 to 5.5%; salicylic acid, 0.45 to 0.55%; resorcinol, 0.45 to 0.55%; mutton oil, 2.7 to 3.3%; alcohol, 2.7 to 3.3%; and white soft paraffin, 60 to 73%.

4. A cream for therapy of dermatitis **characterized in that** the volume ratios of respective ingredients are lightyellow Sophora root, 3%, turmeric 2%; Magnolia bark, 2%; Moutan bark, 2%; Isatis leaf, 1%; Borneo camphor tree (Dryobalanops aromatica Gaertn. f.), 1%; Baikal skullcap 2%; Amur cork tree, 2%; Angelicae Dahuricae root, 1%; lemon, 3% smartweed, 1%; licorice, 0.5%: dimethylsulfoxide, 5%; salicylic acid, 0.5%; resorcinol, 0.5%; mutton oil, 3%; alcohol, 3%; and white soft paraffin, 66.5%.

## Patentansprüche

1. Creme zur Therapie von Dermatitis, **dadurch gekennzeichnet, dass** die Volumenverhältnisse jeweiliger Inhaltsstoffe betragen hellgelbe Sophorawurzel 2,7 bis 3,3%; Kurkuma 1,8 bis 2,2%; Magnoliarinde 1,8 bis 2,2%; Strauchpfingstrosenrinde 1,8 bis 2,2%; Isatisblatt 0,9 bis 1,1 %; Borneo-Kampferbaum (Dryobalanops aromatica Gaertn. f.) 0,9 bis 1,1%; Baikal-Helmkraut 1,8 bis 2,2%; Amur-Korkbaum 1,8 bis 2,2%; Angelicae Dahuricae Wurzel 0,9 bis 1,1%; Zitrone 0 bis 3%; Knöterich 0 bis 1,1%; Süßholz 0 bis 0,55%; Cnidil Rhizoma 0,45 bis 1,1%; Japanische Angelikawurzel 0 bis 0,55%; Salicylsäure 0,45 bis 0,55%; Resorcinol 0,45 bis 0,55%; Hammelöl 2,7 bis 3,3%; Alkohol 2,7 bis 3,3%; und weißes Kohlenwasserstoffgel 63 bis 78%.

2. Creme für die Therapie von Dermatitis, **dadurch gekennzeichnet, dass** die Volumenverhältnisse jeweiliger Inhaltsstoffe betragen hellgelbe Sophorawurzel 3%; Kurkuma 2%; Magnoliarinde 2%; Strauchpfingstrosenrinde 2%; Isatisblatt 1%; Borneo-Kampferbaum (Dryobalanops aromatica Gaertn. f.) 1%; Baikal-Helmkraut 2%; Amur-Korkbaum 2%; Angelicae Dahuricae Wurzel 1 %; Zitrone 3%; Knöterich 1 %; Süßholz 0,5%; Cnidil Rhizoma 0,5%; Japanische Angelikawurzel 0,5%; Salicylsäure 0,5%; Resorcinol 0,5%; Hammelöl 3%; Alkohol 3%; und weißes Kohlenwasserstoffgel 70,5%.

3. Creme für die Therapie von Dermatitis, **dadurch gekennzeichnet, dass** die Volumenverhältnisse jeweiliger Inhaltsstoffe betragen hellgelbe Sophorawurzel 2,7 bis 3,3%; Kurkuma 1,8 bis 2,2%; Magnoliarinde 1,8 bis 2,2%; Strauchpfingstrosenrinde 1,8 bis 2,2%; Isatisblatt 0,9 bis 1,1%; Borneo-Kampferbaum (Dryobalanops aromatica Gaertn. f.) 0,9 bis 1,1%; Baikal-Helmkraut 1,8 bis 2,2%; Amur-Korkbaum 1,8 bis 2,2%; Angelicae Dahuricae Wurzel 0,9 bis 1,1%; Zitrone 0 bis 3%; Knöterich 0 bis 1,1%; Süßholz 0 bis 0,55%; Dimethylsulfoxid 4,5 bis 5,5%; Salicylsäure 0,45 bis 0,55%; Resorcinol 0,45 bis 0,55%; Hammelöl 2,7 bis 3,3%; Alkohol 2,7 bis 3,3%; und weißes Kohlenwasserstoffgel 60 bis 73%.

4. Creme für die Therapie von Dermatitis, **dadurch gekennzeichnet, dass** die Volumenverhältnisse jeweiliger Inhaltsstoffe betragen hellgelbe Sophorawurzel 3%; Kurkuma 2%, Magnoliarinde 2%; Strauchpfingstrosenrinde 2%; Isatisblatt 1%; Borneo Kampferbaum (Dryobalanops aromatika Gaertn. f.) 1%; Baikal-Helmkraut 2%; Amur-Korkbaum 2%; Angelicae Dahuricae Wurzel 1 %; Zitrone 3%; Knöterich 1 %; Süßholz 0,5%; Dimethylsulfoxid 5%; Salicylsäure 0,5%; Resorcinol 0,5%; Hammelöl 3%; Alkohol 3%; und weißes Kohlenwasserstoffgel 66,5.

## Revendications

1. Une crème thérapeutique contre la dermatite **caractérisée en ce que** les proportions en volume d'ingrédients respectifs sont : racine de Sophora jaune clair, 2,7 à 3,3%; curcuma, 1,8 à 2,2%; écorce de magnolia, 1,8 à 2,2%; écorce de pivoine arborescente 1,8 à 2,2%; feuille de pastel, 0,9 à 1,1%; camphrier de Borneo (Dryobalanops aromatica Gaertn.f.), 0,9 à 1,1%; Scutellaire du Baïkal, 1,8 à 2,2%; Phellodendron de l'Amur, 1,8 à 2,2%; racine d'angélique dahurica, 0,9 à 1,1%; citron, 0 à 3%; Renouée du Japon, 0 à 1,1%; réglisse, 0 à 0,55%; rhizome de cnidion (Ligusticum chuanxiong Hort), 0,45 à 1,1%; racine d'angélique du Japon, 0 à 0,55%; acide salicylique, 0,45 à 0,55%; résorcinol, 0,45 à 0,55%; huile de mouton, 2,7 à 3,3%; alcool, 2,7 à 3,3%; et paraffine molle blanche, 63 à 78%.

2. Une crème thérapeutique contre la dermatite **caractérisée en ce que** les proportions en volume d'ingrédients respectifs sont : racine de Sophora jaune clair, 3%; curcuma, 2%; écorce de magnolia, 2%; écorce de pivoine arborescente 2%; feuille de pastel, 1%; camphrier de Borneo (Dryobalanops aromatica Gaertn.f.), 1%; Scutellaire du Baïkal, 2%; Phellodendron de l'Amur, 2%; racine d'angélique dahurica, 1%; citron, 3%; Renouée du Japon, 1%; réglisse, 0,5%; rhizome de cnidion (Ligusticum chuanxiong Hort), 0,5%; racine d'angélique du Japon, 0,5%; acide salicylique, 0,5%; résorcinol, 0,5%; huile de mouton, 3%; alcool, 3%; et paraffine molle blanche, 70,5%.

3. Une crème thérapeutique contre la dermatite **caractérisée en ce que** les proportions en volume d'ingrédients respectifs sont : racine de Sophora jaune clair, 2,7 à 3,3%; curcuma, 1,8 à 2,2%; écorce de magnolia, 1,8 à 2,2%; écorce de pivoine arborescente 1,8 à 2,2%; feuille de pastel, 0,9 à 1,1%; camphrier de Borneo (Dryobalanops aromatica Gaertn.f.), 0,9 à 1,1%; Scutellaire du Baïkal, 1,8 à 2,2%; Phellodendron de l'Amur, 1,8 à 2,2%; racine d'angélique dahurica, 0,9 à 1,1%; citron, 0 à 3%; Renouée du Japon, 0 à 1,1%; réglisse, 0 à 0,55%; diméthylsulfoxide, 4,5 à 5,5%; acide salicylique, 0,45 à 0,55%; résorcinol, 0,45 à 0,55%; huile de mouton, 2,7 à 3,3%; alcool, 2,7 à 3,3%; et paraffine molle blanche, 60 à 73%.

4. Une crème thérapeutique contre la dermatite **caractérisée en ce que** les proportions en volume d'ingrédients respectifs sont : racine de Sophora jaune clair, 3%; curcuma, 2%; écorce de magnolia, 2%; écorce de pivoine arborescente 2%; feuille de pastel, 1%; camphrier de Borneo (Dryobalanops aromatica Gaertn.f.), 1%; Scutellaire du Baïkal, 2%; Phellodendron de l'Amur, 2%; racine d'angélique dahurica, 1%; citron, 3%; Renouée du Japon, 1%; réglisse, 0,5%; diméthylsulfoxide, 5%; acide salicylique, 0,5%; résorcinol, 0,5%; huile de mouton, 3%; alcool, 3%; et paraffine molle blanche, 66,5%.
